# EUROPÄISCHE PATENTSCHRIFT

(11) **EP 1 682 080 B1**
(45) Veröffentlichungstag und Bekanntmachung des Hinweises auf die Patenterteilung: **25.03.2009**
(21) Anmeldenummer: 04765667.3
(22) Anmeldetag: 28.09.2004
(51) Int. Cl.: A61K 8/18

(54) **KAUMASSE ZUR REMINERALISATION VON ZAHNSCHMELZ**
CHEWING GUM FOR THE REMINERALISATION OF TOOTH ENAMEL
MASSE DE GOMME A MACHER POUR LA REMINERALISATION DE L'EMAIL DENTAIRE

(30) Priorität: 17.10.2003 DE 10349050
(43) Veröffentlichungstag der Anmeldung: 26.07.2006
(73) Patentinhaber: MEDERER Süßwarenvertriebs GmbH, 90765 Fürth (DE)
(72) Erfinder: LAHRSOW, Jobst, 97941 Tauberbischofsheim (DE)
(74) Vertreter: Rau, Manfred
(86) Internationale Anmeldenummer: PCT/EP2004/010860
(87) Internationale Veröffentlichungsnummer: WO 2005/037238

(56) Entgegenhaltungen:
- EP-A- 0 497 158
- EP-A- 0 648 108
- EP-A- 0 845 976
- GB-A- 1 516 525

## Beschreibung

Die vorliegende Erfindung betrifft eine Kaumasse zur Remineralisation von Zahnschmelz sowie ein Verfahren zur Herstellung einer solchen Kaumasse.

Bis heute gilt die Anwendung von Fluoriden als Prophylaxemethode der Wahl gegen Karies, da andere Möglichkeiten wie Mundhygiene oder Ernährungslenkung zumeist an der Nachlässigkeit der Menschen scheitern. Dennoch sind die Fluoride nur bedingt für die Gesunderhaltung der Zähne brauchbar, da ihr hauptsächlicher Wirkungsmechanismus, die Förderung der Remineralisation von Zahnschmelz, nur in Anwesenheit von freien Calcium- und Phosphationen möglich ist.

In Perioden, in denen der Zahnschmelz durch die von Mikroorganismen in der Mundhöhle ausgeschiedenen Säuren entkalkt wird, bilden sich an seiner Oberfläche kleine Poren. Aus diesen wandern die Calciumsalze aus der Tiefe des Schmelzes in den Zahnbelag und anschließend in die Mundhöhle aus. Tiefe Poren, in denen die Mineralien Calcium und Phosphat fehlen, bleiben folglich zurück. Sofern diese Entkalkung nicht gestoppt wird, brechen sie dann später bei der eigentlichen Kariesbildung ein.

Bei der natürlicherweise vorkommenden Remineralisation des Zahnschmelzes werden solche Poren durch die im Speichel vorhandenen Calcium- und Phosphationen in der Nähe des Neutralpunktes sehr schnell wie mit einem Korken verschlossen. Tiefer gelegene Schmelzschichten bleiben dabei allerdings mineralarm.

An diesem Punkt setzt die so genannte "forcierte dynamische Remineralisation" ein. Durch Absenken des pH-Wertes bei gleichzeitiger Erhöhung der Mineralkonzentration z. B. mit Hilfe einer sauren, mit Calcium und Phosphaten versetzten Remineralisationslösung oder durch eine entsprechend präparierte Kau- oder Lutschmasse, kann in dem Mundhöhlenmilieu die Konzentration an Mineral um ein Vielfaches erhöht werden, ohne dass die Sättigungsgrenze wesentlich überschritten wird. Bei einer solchen Maßnahme wird der poröse, entkalkte Zahnschmelz mit Mineral durchtränkt. Auf diese Weise wird eine große Menge gelösten Minerals in alle Bereiche der Läsion transportiert.

Nach der Applikation steigt durch die Speichelclearance der pH-Wert in dem Umgebungsmilieu des Zahnes wieder an, gleichzeitig aber sinkt dort die Mineralkonzentration drastisch ab. Dadurch diffundieren aus dem porösen Schmelzbereich sowohl Protonen als auch ein Teil des während der Applikation eingeschleusten Minerals wieder in die Mundhöhle zurück. Wegen der größeren Beweglichkeit der H⁺-Ionen und des nichtlinearen Zusammenhangs zwischen Diffusionszeit und -strecke verarmt der oberflächennahe Bereich schneller an Mineral als die tiefer gelegenen Schichten. In diesen ist das Mineral wegen der größeren Beweglichkeit der Hydroniumionen gefangen und schlägt sich nach deren Abzug - wegen des dadurch bedingten pH-Anstiegs - an den Porenwänden nieder. Auf diese Weise werden durch das zeitliche Konzentrationsprofil, das durch die Applikation vorgegeben ist, sowohl Ort als auch Menge des Mineraleinbaus positiv beeinflusst.

Aus der Praxis sind Fruchtgummis bekannt, die eine Zugabe von Calcium im Bereich von 3 mMol/kg enthalten. Diese Konzentration ist für die Kariesprophylaxe zu gering.

Eine Möglichkeit, die oben genannten zeitlichen Konzentrationsprofile in der Mundhöhle zu erzeugen, bieten Kaumassen insbesondere in Form von Fruchtgummis, die mit Calcium und Phosphat angereichert sind. Derartige Kaumassen sind hinsichtlich der Konzentrationen von Calcium und Phosphat in der Patentschrift EP 0 648 108 B1 in allgemeiner Form neben anderen Ausführungsbeispielen beschrieben. Dort wird vorgeschlagen, eine Konzentration an Calcium in der Kaumasse zwischen 200 mMol/kg und 800 mMol/kg und die für Phosphat im Bereich von 50 mMol/kg und 400 mMol/kg einzustellen. Ein Herstellungsverfahren für eine derartige Kaumasse ist nicht offenbart. Nachteilig ist, dass die hohe Calciumkonzentration in der Kaumasse die Transparenz und Homogenität der Kaumasse beeinträchtigt.

In der US 2001/0033831 A1 wird die Zugabe von α-Tricalciumphosphat in remineralisierenden Kaugummi vorgeschlagen. Zwar ist α-Tricalciumphosphat besser löslich als die anderen neutralen und basischen Calciumphosphate, eine effektive über die des Speichels hinausgehende Remineralisationswirkung ist aber nicht zu erwarten.

Die Implementierung von Calcium und Phosphat aus den Calciumsalzen der Frucht- bzw. Carbonsäuren und Phosphaten liefert nicht die gewünschten Ergebnisse. Zwar ist aus der US 5,015,628 ein Produkt bekannt, in dem Calciumphosphat zugegeben ist. Die erforderliche Konzentration von Calcium, das für den Remineralisationsprozess verfügbar ist, ist aber zu gering.

Ein entsprechendes Produkt befindet sich auch nicht auf dem Markt. Die Probleme bei der Herstellung sind durch den Stand der Technik nicht gelöst, insbesondere nicht für Kaumassen auf Gelatinebasis.

Bekanntermaßen beeinflussen Ca-Ionen das Quellverhalten von Gelatine negativ. Calcium kann zur Trübung der für Fruchtgummis verwendeten Gelatine bis hin zu ihrer Gerinnung führen. Wenn das Calcium in üblicher Weise zusammen mit Zucker, Farbstoffen, Aromen etc. in Form von Salzen zugegeben wird, liegt die Grenze zur Trübung der Gelatine etwa bei 5 mMol/kg. Für verkaufsfähige Produkte wird jedoch stets eine glasklare, homogene Konsistenz der Gelatine bevorzugt.

Demzufolge liegt der Erfindung die Aufgabe zu Grunde, ein Verfahren zur Herstellung einer transparenten und homogenen Kaumasse, zur Remineralisation von Zahnschmelz bereitzustellen, die eine gute Remineralisationswirkung aufweist. Ferner liegt der Erfindung die Aufgabe zu Grunde, eine Kaumässe bereitzustellen, die transparent und homogen ist und gleichzeitig eine gute Remineralisationswirkung aufweist.

Diese Aufgaben werden von einem Verfahren mit den Merkmalen des Anspruchs 1 und von einer Kaumasse mit den Merkmalen des Anspruchs 7 gelöst.

Weil bei dem erfindungsgemäßen Verfahren zur Herstellung einer Kaumasse zur Remineralisation von Zahnschmelz folgende Schritte vorgesehen sind:
a. Ansatz einer wässrigen Lösung von wenigstens einem nach dem Lebensmittelgesetz zugelassenen Säuerungsmittel, z. B. aus der Gruppe der Carbonsäuren insbesondere der Fruchtsäuren, und Phosphorsäure;
b. Zugabe eines reaktiven Calciumspenders, z. B. Calciumhydroxid;
c. Zugabe von Verdickungsmittel, z. B. von in gemahlener oder gequollener Form vorliegender Gelatine, zu der Lösung;
d. inniges Mischen der Komponenten;
e. Ausformen der Masse und Trocknen z. B. in Maisstärke. erhält man eine transparente, und homogene Kaumasse mit den gewünschten Eigenschaften in der Kariesprophylaxe und zur Beeinflussung von Initialkaries. Das nach diesem Verfahren hergestellte Produkt zeichnet sich durch eine besonders gute Transparenz und Homogenität aus.

Das Geliermittel kann einen Teil der Geschmacks- und Hilfsstoffe enthalten. Diese können aber auch separat zu der Lösung zugegeben werden.

Die Zugabe der Phosphorsäure kann auch zwischen Schritt (b) und (c) erfolgen. Ebenso ist es möglich, das Geliermittel grob gemahlen zur Lösung zuzugeben und dort quellen zu lassen.

Weil bei einer Kaumasse nach Anspruch 7 vorgesehen ist, dass der Calciumgehalt zwischen 50 mMol/kg und 150 mMol/kg von (2,3 g/kg bis 7,0 g/kg) bezogen auf das Fertigprodukt beträgt, ist ein einfach zu fertigendes und langzeitstabiles Produkt verfügbar, das dennoch eine gute Wirkung in vivo aufweist. Diese Wirkung wird dadurch erreicht, dass die lokale Konzentration an der Zahnoberfläche durch die anhaftende Kaumasse besonders groß ist und ein speichelbedingter Austrag der Calcium- und Phosphationen im Kontaktbereich zwischen der Kaumasse und dem Zahn nicht in wesentlichem Maße erfolgt. Insbesondere kann bei dieser Calciumkonzentration der Phosphorgehalt zwischen 15 mMol/kg und 500 mMol/kg betragen.

Für eine gute Funktion der forcierten Remineralisation sollen das Calcium und das Phosphat in dem Fruchtgummi möglichst vollständig gelöst vorliegen, cl. h. es sollte möglichst in Ionenform und/oder kolloidal, aber nicht kristallin als Salz vorliegen.

Das Verfahren ist darauf ausgelegt, sich möglichst gut in den herkömmlichen Arbeitsablauf bei der Produktion von Fruchtgummi einzufügen. Dabei lässt man das jeweilige Verdickungsmittel, z. B. die Gelatine, in einem Teil der Flüssigkeit, die auch einen Teil der Hilfs- und Zusatzstoffe enthalten kann, quellen. Das gequollene Geliermittel, im Folgenden "Teil 1" genannt, wird dann mit den restlichen Zutaten (das restliche Wasser, der Zucker, die Säure, Aroma -und Farbstoffe sowie das Calcium und Phosphat), im Folgenden "Teil 2" genannt, unter Vermeidung von Luftblasen sorgfältig zur Fruchtgummimasse gemischt, geformt, in Maisstärkeformen getrocknet und dann weiter verarbeitet z. B. gewachst etc..

Werden z. B. Calcium und Phosphat in konventioneller Weise aus Salzen in die Lösung Teil 2 implementiert, so klumpt die Gelatine in allen Fällen, besonders stark beim Calcium-Lactat-Milchsäure-Phosphatpuffer.

Die Transparenz und Homogenität des Produktes sowie dessen Remineralisationsvermögen kann zusätzlich durch die geeignete Mischung verschiedener Säuerungsmittel als Komponente für Teil 2 gesteuert werden. Insbesondere lassen sich die relevanten Eigenschaften der Kaumassen durch Variation der Anteile an Säuren mit unterschiedlichem Komplexierungsvermögen von Calcium steuern.

Beispielsweise führt die Zumischung von Äpfelsäure oder Zitronensäure zu einem Fruchtgummi, der auf Brenztraubensäure konzipiert ist, zu besonders klaren und in der Säure angenehmen Fruchtgummimischung mit guter Wirksamkeit.

### Rezepturen und Beispiele

In den folgenden Beispielen wird zum Quellen reines Wasser verwendet. Die Quellzeit der Blattgelatine variiert zwischen 1 Stunde und 24 Stunden bei Temperaturen zwischen 37°C und 60°C (Teil 1).

Bestandteile aus Teil 2, die das Quellverhalten der Gelatine nicht stören, können auch in den Teil 1 gegeben werden.

Ausgangsstoffe sind die in der Flüssigkeitsmenge des Teil 2 gelösten Carbonsäuren und Phosphorsäure in der für die forcierte Remineralisation geeigneten Konzentration und als reaktiver Calciumspender Calciumoxid, Calciumhydroxid oder Calciumcarbonat oder eine Mischung davon. Die Menge an Phosphorsäure richtet sich nach der gewünschten Phosphatkonzentration des Fertigproduktes und liegt im Bereich von 15 mMol/kg bis 500 mMol/kg (1.4 g/kg bis 48 g/kg) bezogen auf das Fertigprodukt. In den angegebenen Beispielen wird ein Gehalt von 70 mMol/kg Phosphat nicht überschritten, obwohl ein höherer Phosphatgehalt der Wirkung zusätzlich dienlich wäre. Diese Beschränkung berücksichtigt das zum Zeitpunkt der Anmeldung gültige deutsche Lebensmittelrecht. Die Frucht- oder Carbonsäurekonzentration richten sich nach dem gewünschten Calciumgehalt, pH-Wert und dem Geschmack der Kaümasse.

Bezogen auf das Fertigprodukt liegt der Calciumgehalt zwischen 30 mMol/kg und 600 mMol/kg (1.2 g/kg und 24 g/kg). Die Neutralisationsreaktion zeigt eine starke positive Wärmetönung, so dass in der Regel auf eine zusätzliche Erwärmung zur Beschleunigung des Reaktionsablaufes verzichtet werden kann. Bei den in den Beispielen angegebenen Konzentrationen an Säuerungsmitteln, Phosphat und Calcium sind diese Lösungen über längere Zeit stabil.

Die im Folgenden aufgeführten Beispiele sind mögliche Realisationen der Erfindung.

Die folgenden Stoffe wurden als Zusätze verwendet:

| | |
|---|---|
| Gelatine: | Blattgelatine Dr. Oetker; |
| Ca(OH)₂: | Merck 2047; |
| Orthophosphorsäure 85%: | Merck 1.00563; |
| Zitronensäure: | Merck 8.18707; |
| Äpfelsäure: | Merck 1.00382; |
| Brenztraubensäure: | Merck 8.20170 |

Die Ausbeute der nachfolgenden Rezepte ergibt ca. 65 g.

Die gesamte Gelatine quillt stets zunächst in 15 ml Aqua dest. für 12 Stunden bei ca. 50°C.

Die Ca-Konzentration im Produkt liegt bei 200 mMol/kg, die Phosphatkonzentration bei 70 mMol/kg.

### Beispiel 1:

Teil 1: 6.7 g Gelatine in 15 ml Aqua dest.
Teil 2: 10 ml Äpfelsäure (1.5 Mol/l) + 10 ml Zitronensäure (1.0 Mol/l) + 0.3 ml Phosphorsäure + 0.9 g Calciumhydroxid + 20 g Haushaltszucker

Die drei Säuren werden gemischt und das Calciumhydroxid wird unter Rühren zugegeben. Nach vollständigem Auflösen wird der Zucker in der Lösung bei schwachem Erwärmen aufgelöst und die warme Gelatinelösung in die Lösung Teil 2 eingerührt. Es folgen das Einbringen in die Maismehlformen und ein ca. 20 bis 48 stündiges Trocknen.

### Beispiel 2:

Teil 1: 6.7 g Gelatine in 15 ml Aqua dest.
Teil 2: 20 ml Zitronensäure (1.5 Mol/l) + 0.3 ml Phosphorsäure + 0.7 g Calciumhydroxid + 20 g Haushaltszucker

### Verarbeitung wie Beispiel 1

### Beispiel 3:

Teil 1: 10 g Gelatine in 15 ml-Aqua dest.
Teil 2: 20 ml Brenztraubensäure (1 Mol/1) + 0.3 ml Phosphorsäure + 0.7 g Calciumhydroxid + 20 g Haushaltszucker

### Verarbeitung wie Beispiel 1

### Beispiel 4:

Teil 1: 8 g Gelatine in 15 ml Aqua dest.
Teil 2: 10 ml Äpfelsäure (1.5 Mol/l) + 10 ml Brenztraubensäure (1.5 Mol/l) + 0.3 ml Phosphorsäure + 0.7 g Calciumhydroxid + 20 g Haushaltszucker

### Verarbeitung wie Beispiel 1

### Beispiel 5:

Teil 1: 8 g Gelatine in 15 ml Aqua dest.
Teil 2: 10 ml Brenztraubensäure (1.5 Mol/l) + 10 ml Zitronensäure (1.5 Mol/l) + 0.3 ml Phosphorsäure + 0.8 g Calciumhydroxid + 20 g Haushaltszucker

### Verarbeitung wie Beispiel 1

### Nachweis der Wirksamkeit

Die remineralisierende Wirkung der beschriebenen Fruchtgummis wurde in einem in vitro-Experiment nachgewiesen. Um eine Vorstellung von der Bedeutung der gemessenen Zahlen zu erhalten und die Relationen zwischen in vivo- und in vitro-Experimenten zu erkennen, sind in den letzten sechs Zeilen der Tabelle Werte zur Mineraleinlagerung aus einem in situ- und parallel dazu in vitro durchgeführten Experiment über die Remineralisationswirkung von Fluoridzahnpasten eingetragen.

Die in vitro und in situ gemessenen Werte bestätigen sich gegenseitig, wodurch die Übertragbarkeit der experimentellen Designs gegeben ist.

Die Ergebnisse zeigen einen deutlichen Mineraleinbau bei den mit remineralisierenden Fruchtgummis behandelten Proben. Die Menge an eingebautem Mineral variiert mit der Calcium-Komplexierung der einzelnen Fruchtsäuren.

Die im vorliegenden Experiment gefundenen Einbauraten bezogen auf jeweils eine Behandlung sind beim remineralisierenden Fruchtgummi bis um den Faktor 4 größer als die bei der Zahnpflege mit einer Fluoridzahnpaste.

**In-vitro Remineralisation kariöser poröser Hydroxylapatit-Sinterkörper mit Fruchtgummi im Vergleich zu einer marktgängigen Zahnpasta**

| Säure | pH | Einlagerung absolut in µg | | Einlagerungsrate in µg/Applikation | |
|---|---|---|---|---|---|
| | | Versuch 1 | Versuch 2 | Versuch 1 | Versuch 2 |
| *in vitro:* | | | | | |
| Äpfelsäure | 4,4 | 680 | 650 | 136 | 130 |
| Citronensäure | 4,2 | 310 | 420 | 62 | 84 |
| Brenztraubensäure | 4,4 | 1760 | 2090 | 352 | 418 |
| Milchsäure | 4,4 | 1960 | --- | 392 | ---- |
| Äpfelsäure/Citronensäure | 4,2 | 430 | 870 | 86 | 174 |
| Kontrolle (Speichel) | 6,5 | -300 | -50 | -60 | -10 |
| Kontrolle (nur Placebo- | 4,3 | | -80 | | -16 |
| Fruchtgummi ohne Ca/PO₄ | | | | | |
| | | | | | |
| *in vitro:* | | | | | |
| MFP-Zahnpasta | | | | 101 | |
| Plazebozahnpasta | | | | -7 | |
| | | | | | |
| *in situ:* | | | | | |
| MFP-Zahnpasta | | | | 79 | |
| Plazebozahnpasta | | | | 38 | |

## Patentansprüche

1. Verfahren zur Herstellung einer Kaumasse zur Remineralisation von Zahnschmelz mit folgenden Schritten:
a. Ansatz einer wässrigen Lösung von wenigstens einem lebensmittelgeeigneten Säuerungsmittel;
b. Zugabe eines reaktiven Calciumspenders;
c. Zugabe der Lösung zu einem Verdickungsmittel, insbesondere zu in gemahlener oder gequollener Form vorliegender Gelatine;
d. inniges Mischen der Komponenten zu einer Masse;
e. Ausformen der Masse und Trocknen;
wobei in wenigstens einem der Schritte a, b oder c Phosphorsäure zugegeben wird.

2. Verfahren nach Anspruch 1, **dadurch gekennzeichnet, dass** zusätzlich folgender Schritt vorgesehen ist:
Mischung verschiedener Säuerungsmittel als Edukt für Verfahrensschritt a).

3. Verfahren nach einem der vorhergehenden Ansprüche, **dadurch gekennzeichnet, dass** das Säuerungsmittel im Schritt a) rein oder als Mischung ausgewählt ist aus der Gruppe, die folgendes umfasst:
- Carbonsäuren, darunter insbesondere
- Milchsäure
- Fruchtsäuren, insbesondere
- Brenztraubensäure
- Zitronensäure
- Äpfelsäure.

4. Verfahren nach einem der vorhergehenden Ansprüche, **dadurch gekennzeichnet, dass** zusätzlich folgender Schritt vorgesehen ist:
Zumischung einer stark Calcium-komplexierenden Säure zu einer Lösung, die im Verfahrensschritt a) mit einer weniger stark Calcium-komplexierenden Säure als Säuerungsmittel hergestellt ist.

5. Verfahren nach Anspruch 4, **dadurch gekennzeichnet, dass** die stark Calcium-komplexierende Säure Äpfelsäure oder Zitronensäure ist und die weniger stark Calcium-komplexierende Säure Brenztraubensäure ist.

6. Verfahren nach einem der vorhergehenden Ansprüche, **dadurch gekennzeichnet, dass** der Calciumspender als reine Verbindung oder Mischung ausgewählt ist aus der Gruppe, die folgendes umfasst:
- Calciumoxid
- Calciumhydroxid
- Calciumcarbonat.

7. Kaumasse zur Remineralisation von Zahnschmelz,
a. welche
i. nach einem Verfahren gemäß Anspruch 1 hergestellt ist, und
ii. Calcium und Phosphor aufweist, wobei
b. der Calciumgehalt zwischen 50 mMol/kg und 150 mMol/kg beträgt, und
c. der Phosphorgehalt zwischen 15 mMol/kg und 500 mMol/kg beträgt.

## Claims

1. Method of producing a chewable mass for remineralization of tooth enamel, including the following steps:
a. preparing an aqueous solution of at least one acidifying agent that is suitable as a foodstuff;
b. adding a reactive calcium source;
c. adding the solution to a thickener, in particular to gelatin in a ground or swollen state;
d. thoroughly mixing all components to form a mass;
e. shaping and drying said mass,
wherein phosphoric acid is added in at least one of the steps a, b or c.

2. Method according to claim 1, **characterized in that** there is additionally provided the following step:
mixing various acidifying agents as a reactant for step a).

3. Method according to one of the preceding claims, **characterized in that** said acidifying agent of step a) is pure or a mixture that has been selected from the group comprising:
- carboxylic acids, including in particular
- lactic acid
- fruit acids, in particular
- pyruvic acid
- citric acid
- malic acid.

4. Method according to one of the preceding claims, **characterized in that** there is additionally provided the following step:adding a strong calcium-complexing acid to a solution which has been produced in step a) using a calcium-complexing acid as an acidifying agent that is less strong.

5. Method according to claim 4, **characterized in that** said strong calcium-complexing acid is malic acid or citric acid, and said calcium-complexing acid, which is less strong, is pyruvic acid.

6. Method according to one of the preceding claims, **characterized in that** said calcium source is a pure compound or a mixture that is selected from the group comprising:
- calcium oxide
- calcium hydroxide
- calcium carbonate.

7. Chewable mass for remineralization of tooth enamel
a. which
i. is produced using a method according to claim 1 and
ii. includes calcium and phosphor, wherein
b. the calcium content amounts to between 50 mMol/kg and 150 mMol/kg, and
c. the phosphor content amounts to between 15 mMol/kg and 500 mMol/kg.

## Revendications

1. Procédé de préparation d'une masse de gomme à mâcher pour la reminéralisation de l'émail dentaire, comprenant les étapes suivante :
a. préparation d'une solution aqueuse d'au moins un agent acidifiant approprié pour les produits alimentaires ;
b. addition d'un donneur de calcium réactif ;
c. addition de la solution à un agent épaississant, notamment à une gélatine se présentant sous forme broyée ou gonflée ;
d. mélange intime des composants pour former une masse ;
e. formage de la masse et séchage ;
où, dans au moins une des étapes a, b ou c, de l'acide phosphorique est ajouté.

2. Procédé selon la revendication 1, **caractérisé en ce que** l'étape suivante est en plus prévus :
mélange de différents agents acidifiants à titre de réactif pour l'étape de procédé a).

3. Procédé selon l'une quelconque des revendications précédentes, **caractérisé en ce que** l'agent acidifiant à l'étape a) sous forme pure ou sous forme de mélange est choisi dans le groupe qui comprend les éléments suivants :
- acides carboxyliques, dont notamment
- acide lactique
- acides de fruits, notamment
- acide pyruvique
- acide citrique
- acide malique.

4. Procédé selon l'une quelconque des revendications précédentes, **caractérisé en ce que** l'étape suivante est en plus prévue :
addition d'un acide complexant fortement le calcium à une solution qui est préparée à l'étape de procédé a) avec un acide complexant moins fortement le calcium à titre d'agent acidifiant.

5. Procédé selon la revendication 4, **caractérisé en ce que** l'acide complexant fortement le calcium est l'acide malique ou l'acide citrique et l'acide complexant moins fortement le calcium est l'acide pyruvique.

6. Procédé selon l'une quelconque des revendications précédentes, **caractérisé en ce que** le donneur de calcium sous forme de composé pur ou de mélange est choisi dans le groupe, qui comprend les éléments suivants :
- oxyde de calcium
- hydroxyde de calcium
- carbonate de calcium.

7. Masse de gomme à mâcher pour la reminéralisation de l'émail dentaire,
a. qui
i. est préparée d'après un procédé selon la revendication 1, et
ii. comprend du calcium et du phosphore, où
b. la teneur en calcium atteint entre 50 mMol/kg et 150 mMol/kg, et
c. la teneur en phosphore atteint entre 15 mMol/kg et 500 mMol/kg.
